(19) Europäisches Patentamt
European Patent Office
Office européen des brevets

(11) Veröffentlichungsnummer : **0 341 516 B1**

# (12) EUROPÄISCHE PATENTSCHRIFT

(45) Veröffentlichungstag der Patentschrift : **18.03.92 Patentblatt 92/12**

(51) Int. Cl.$^5$ : **C08G 18/08,** C08K 5/09

(21) Anmeldenummer : **89107742.2**

(22) Anmeldetag : **28.04.89**

## (54) Stabilisierte Polyisocyanate.

(30) Priorität : **11.05.88 DE 3816118**

(43) Veröffentlichungstag der Anmeldung : **15.11.89 Patentblatt 89/46**

(45) Bekanntmachung des Hinweises auf die Patenterteilung : **18.03.92 Patentblatt 92/12**

(84) Benannte Vertragsstaaten : **BE DE ES FR GB IT NL**

(56) Entgegenhaltungen :
**EP-A- 0 127 356**
**EP-A- 0 155 559**
**DE-A- 2 436 741**
**DE-A- 2 549 925**

(73) Patentinhaber : **BAYER AG**
**W-5090 Leverkusen 1 Bayerwerk (DE)**

(72) Erfinder : **Pedain, Josef, Dr.**
**Haferkamp 6**
**W-5000 Koeln 80 (DE)**
Erfinder : **Kahl, Lothar, Dr.**
**Schützheiderweg 27**
**W-5060 Bergisch Gladbach 2 (DE)**
Erfinder : **Weber, Karl-Arnold, Dr.**
**Rothwald-Strasse 13**
**W-7291 Betzweiler/Walde II (DE)**

EP 0 341 516 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Polyisocyanate, die für Lacke und Beschichtungen eingesetzt werden, sind im allgemeinen Reaktionsprodukte aus Diisocyanaten. Diese werden durch Urethanisierung, Harnstoffbildung, Allophanatisierung, Biuretisierung, Trimerisierung und Dimerisierung und ähnlichen Reaktionsmögichkeiten modifiziert. Die auf diese Weise modifizierten Polyisocyanate enthalten oft kein oder nur sehr wenig freies Ausgangsdiisocyanat. Sie enthalten aber noch freie NCO-Gruppen, die nach wie vor eine hohe Reaktivität haben, die für eine spätere Anwendung genutzt werden soll. Sie müssen aber über lange Zeiträume unverändert beständig sein und gehandelt werden können.

Es ist deshalb bereits vorgeschlagen worden, modifizierte Polyiscoyanate durch Zusätze von bestimmten Verbindungen, die im allgmeinen sauren Charakter haben, zu stabilisieren.

Ein Stabilisator soll mehrere Funktionen erfüllen:

– er soll Reaktionsbeschleuniger und Katalysatoren, die vorher gegebenenfalls bei Umsetzungen benutzt wurden oder mit Reaktionspartnern wie z.B. Polyethern oder Polyestern ungewollt aber zwangsläufig eingeschleppt wurden, binden und wirkungslos machen;

– er soll verhindern, daß das Polyisocyanat durch Licht oder ähnliche äußere Einflüsse nachteilig verändert wird und darf natürlich selbst keine Veränderungen wie Verfärbungen hervorrufen; er soll erreichen, daß das Polyisocyanat unverändert bleibt und seine hohe Reaktivität behält;

– er soll bei der nachfolgenden Anwendung des Polyisocyanats dafür sorgen, daß die Reaktivität gegenüber Reaktionspartnern wie Polyalkoholen oder gegebenenfalls blockierten Polyaminen reproduzierbar ist, d.h. er soll gleichzeitig ein Katalysator für nachfolgende Umsetzungen sein.

Daraus geht hervor, daß in der vorliegenden Anmeldung unter dem Begriff "Stabilisator" nicht die Polyurethanschaumstabilisatoren gemeint sind, die seit langem in der Isocyanatchemie eine große Rolle spielen und oberflächenaktive Mittel z.B. auf Polysiloxan-Basis sind.

Die Synthese von Polyisocyanaten, die für Beschichtungszwecke geeignet sind oder die für Verklebungen und als Dichtstoffe eine Rolle spielen ist durch zahlreiche Veröffentlichungen vorbeschrieben. Als Beispiele für Übersichten seien genannt:

1. G.W. Becker und D. Braun, Kunststoff Handbuch Band 7 "Polyurethane", Herausgeber G. Oertel, Carl-Hanser-Verlag, München, Wien 1983, Seiten 540-610
2. J.H. Saunders und K.C. Frisch, "High Polymers", Verlag Interscience Publishers, New York 1962, Vol. XVI
3. H. Kittel, Lehrbuch der Lacke und Beschichtungen, Verlag W.A. Colomb Berlin 1973, Band I, Teil 2
4. K.C. Frisch, P. Kordomenos, American Chemical Society Symposium, 285, "Applied Polymer Science", Seite 985, 2. Auflage, Washington 1985.

Die Modifizierung von Polyisocyanaten, die für Beschichtungsmittel, Dichtstoffe und Klebstoffe Verwendung finden, mit Zusätzen von bestimmten Verbindungen aus der Klasse von Säuren oder Substanzen mit saurem Charakter zum Zwecke einer Stabilisierung und/oder zur Reaktivitätskontrolle gegenüber bestimmten Reaktionspartnern wie Polyolen oder gegebenenfalls blockierten Polyaminen ist mehrfach vorbeschrieben. In der EP-A 155 559 werden beispielweise in Isocyanuratpolyisocyanaten enthaltene basische Katalysatoren durch Säuren wie Phosphorsäure, Dodecylbenzolsulfonsäure, Monochloressigsäure, Monofluoressigsäure oder Benzoylchlorid beseitigt und dadurch die Polyisocyanate stabilisiert. In EP-A 239 834 und EP-A 254 177 wurden zu Urethangruppen enthaltenden Polyisocyanaten Verbindungen zugesetzt wie Ameisensäure, Essigsäure, Mono-, Di- und Trichloressigsäure, Oxalsäure, Malonsäure, Maleinsäure, Fumarsäure, Benzoesäure, Mono-, Di- und Trichlorbenzoesäure, Salicylsäure, Toluolsulfonsäure, Xylolsulfonsäure u.a. Durch diesen Zusatz wird erreicht, daß die genannten Polyisocyanate gegenüber Reaktionspartnern wie Polyaldiminen, mit denen sie bei Zutritt von Feuchtigkeit abreagieren, eine konstante Reaktivität besitzen, da die Zusätze auch die Hydrolysegeschwindigkeit der Polyaldimine beeinflussen.

Die genannten und bekannten Zusätze erfüllen allerdings die vorher genannten Forderungen an einen Stabilisator nur teilweise. Sie haben zum Teil sogar nachteilige Wirkungen u.a. verursachen sie Verfärbungen des Polyisocyanats oder verschlechtern die Beständigkeit der daraus hergestellten Beschichtungs-, Dichtungs- und Klebstoffmassen gegenüber hydrolytischen Einflüssen. Ihre Wirkung kann bei Lagerung verloren gehen, die Eigenschaften der zu stabilisierenden Polyisocyanate können sich deshalb verändern. Deshalb wird auch vorgeschlagen, noch weitere Stabilisatoren wie Phenothiazine, sterisch gehinderte Phenole usw. zusätzlich mitzuverwenden.

Aufgabe der vorliegenden Erfindung war es deshalb, modifizierte Polyisocyanate mit verbesserter Lagerstabilität zur Verfügung zu stellen.

Diese Aufgabe konnte überraschend gelöst werden durch das Auffinden der Wirksamkeit von speziellen Carbonsäuren, die in kleinen Mengen zu dem modifizierten Polyisocyanat zugesetzt werden.

Gegenstand der Erfindung sind deshalb Polyisocyanate, die eine Carbonsäure der allgemeinen Formel

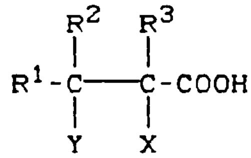

enthalten, worin
$R^1$, $R^2$ und $R^3$ Wasserstoff oder $C_1$-$C_5$-Alkyl und
X und Y Wasserstoff, Chlor oder Methyl bedeuten, mit der Maßgabe, daß
X = Cl, wenn Y = H oder $CH_3$ bzw. Y = Cl, wenn X = H oder $CH_3$.

Gegenstand der Erfindung sind auch die derart stabilisierten Polyisocyanate und ihre Verwendung für Beschichtungs-, Dichtungs-und Klebstoffbindemittel.

Die erfindungsgemäß zu verwendenden Chlorcarbonsäuren sind bekannt. Genannt seien 3-Chlor-2,2-dimethyl-propansäure, 2-Chlorbutansäure und 2- und 3-Chlorpropansäure. 2-Chlorpropansäure ist die besonders bevorzugte Säure.

Die genannten Säuren werden in einer Menge von 0,0001 bis 1 Gew.-%, bevorzugt von 0,001 bis 0,1 Gew.-% (bezogen auf Polyisocyanat) zu dem modifizierten Polyisocyanat zugesetzt bzw. in geeigneter Weise homogen eingearbeitet. Es ist aber auch möglich die erfindungsgemäß zu verwendenden Säuren schon während der Herstellung des modifizierten Isocyanats zuzusetzen.

Erfindungsgemäß können beliebige modifizierte Polyisocyanate, die neben freien NCO-Gruppen noch Urethangruppen und/oder Allophanatgruppen und/oder Harnstoffgruppen und/oder Biuretgruppen und/oder Isocyanuratgruppen und/oder Uretdiongruppen enthalten, stabilisiert werden.

Beispielhaft genannt seien Polyisocyanate aus den Diisocyanaten 2,4- und 2,6-Toluylendiisocyanat, 2,4′- und 4,4′-Diphenylmethandiisocyanat, 1,6-Hexamethylendiisocyanat, 1-Isocyanato-3,3,5-trimethyl-5-isocyanatomethylcyclohexan, 2,4′- und 4,4′-Dicyclohexylmethandiisocyanat und beliebigen Gemischen dieser Diisocyanate. Weitere Diisocyanate und Modifizierungsprodukte dieser Diisocyanate sind z.B. in DE-A 2 637 115 erwähnt, d.h. also Polyisocyanate bzw. NCO-Vorpolymere, die mit Wasser und einem Härtergemisch aus den Verbindungen der allgemeinen Formeln

$$H_2N\text{-}R\text{-}NH_2 \quad (A)$$
$$H_2N\text{-}R\text{-}N{=}R_1 \quad (B)$$
$$R_1{=}N\text{-}R\text{-}N{=}R_1 \quad (C),$$

Polyurethanharnstoffe bilden.

In diesen Formeln bedeuten:
R einen divalenten aliphatischen, cycloaliphatischen oder araliphatischen Rest
$R_1$ einen aliphatischen oder cycloaliphatischen Rest, wie er durch Entfernung des Sauerstoffs aus einem Keton oder Aldehyd entsteht,
wobei die Molverhältnisse

$$\frac{A}{B + C} = 1{:}20 \text{ bis } 1{:}3$$
$$\frac{B}{C} = 1{:}2 \text{ bis } 2{:}1 \text{ sowie}$$
$$\frac{A + B + C}{H_2O} = 1{:}1{,}4 \text{ bis } 1{:}20.$$

eingehalten werden.

Die in DE-A 2 637 115, DE-A 3 011 711, EP-A 3569 beschriebenen modifizierten Polyisocyanate eignen sich in besonderer Weise für eine Stabilisierung. Auch die Polyisocyanate, die in EP-A 0 254 177 und EP-A 0 239 834 beschrieben sind und für Beschichtungszwecke Verwendung finden, lassen sich sehr gut stabilisieren bzw. in ihrer Reaktivität gegenüber Polyaldiminen reproduzierbar einstellen.

Weitere modifizierte Polyisocyanate, die sich erfindungsgemäß vorteilhaft stabilisieren lassen, sind in DE-A 2 641 448 beschrieben.

Das erfindungsgemäß stabilisierte Produkt zeigt gegenüber dem Stand der Technik bemerkenswerte Vorteile:

– sie haben eine verbesserte Lagerstabilität d.h. vor allem Farbzahl, Viskosität und NCO-Gehalt der Polyisocyanate bleiben über lange Zeit konstant;

– sie lassen sich mit Reaktionspartnern leichter und reproduzierbar verarbeiten, d.h. insbesondere in Kombination mit Polyaldiminen (z.B. gemäß EP-A 254 117) und Polyketiminen (z.B. gemäß DE-A 2 637 115) lassen sich mit den erfindungsgemäßen Verfahrensprodukten wiederholbar Beschichtungen mit gleichen Eigenschaften herstellen.

Beispiel 1

6000 g (3,53 Mol) Hydroxylpolyester aus Adipinsäure, Hexandiol-1,6 und Neopentylglykol werden im Vakuum bei 120° C entwässert und mit 1290 g (7,41 Mol) Toluylendidisocyanat (80 % 2,4-, 20 % 2,6-Isomeres) vermischt und in 30 Minuten bei 90° C zur Reaktion gebracht. Nun werden 320 g eines Mischtrimerisates aus 3 mol 2,4-Toluylendiisocyanat und 2 Mol 1,6-Hexamethylendiisocyanat zugefügt (5 Mol-% berechnet auf gesamte Isocyanatkomponente), das Reaktionsgemisch 30 Minuten bei 90°C nachreagieren gelassen, auf 60° C abgekühlt und mit 1900 g Ethylacetat verdünnt. NCO-Gehalt des erhaltenen Polyisocyanats: 3,45 Gew.-% der ca. 80 %igen Lösung.

Bei einer Wiederholung des Versuchs wurde ein NCO-Gehalt von 3,62 Gew.-% bei einer 80 %igen Lösung gemessen.

Beispiel 2

6039 g eines Hydroxylpolyesters aus Adipinsäure, Neopentylglykol und Hexandiol-1,6 mit dem mittleren Molgewicht 1700 werden mit 1655 g 1-Isocyanato-3,3,5-trimethyl-5-isocyanatomethylcyclohexan vermischt und bei 100° C in 5 Stunden unter Rühren zu einem NCO-Prepolymeren umgesetzt. Nun werden 314 g des Mischtrimerisats wie in Beisiel 1 beschrieben eingemischt und noch einige Minuten bei 60° C gerührt. Man löst ca. 80 %ig in Ethylacetat und erhält eine Lösung eines Polyisocyanats mit einem NCO-Gehalt von 3,28 % und 4200 mPas.

Beispiel 3

Eine Mischung aus 1700 g 3,3,5-Trimethyl-5-aminomethylcyclohexylamin (IPDA), 130 g Wasser und 4170 g Methylethylketon wird 2 Stunden unter Rückfluß gekocht. Nach dem Abkühlen ist der Härter gebrauchsfertig. Er enthält freie Aminogruppen und Ketimingruppen.

Beispiel 4

Der Versuch von Beispiel 1 wird wiederholt mit folgendem Unterschied: Nach der Entwässerung des Polyesters gibt man 200 ppm (bezogen auf Gesamtansatz ohne Lösemittel) 2-Chlorpropionsäure zu, die man gut untermischt und verfährt danach wie in Beispiel 1 angegeben d.h. Umsetzung mit Toluylendiisocyanat und Abmischung mit dem Mischtrimerisat aus Toluylendiisocyanat und Hexamethylendiisocyanat.

Die 80 %ige Lösung in Ethylacetat hat einen NCO-Gehalt von 3,52 Gew.-%.

Beispiele 5-17

In diesen Beispielen wird ein erfindungsgemäßer Stabilisator in seiner Wirksamkeit mit Stabilisatoren vom Stand der Technik vergleichen. Die Stabilisatoren werden der Polyisocyanatlösung gemäß Beispiel 1 bzw. Beispiel 4 (bei Nr. 17) bei ca. 25° C in einer Menge von 20 ppm (bezogen auf lösungsmittelfreies Produkt) zugesetzt und durch Rühren vermischt, bis eine klare homogene Flüssigkeit entstanden ist.

Untersucht werden die Veränderungen, die bei NCO-Gehalt, Farbe und Reaktivität während der Lagerung auftreten. Die Lagerung der Proben erfolgt in farblosen Glasflaschen unter Feuchtigkeitsausschluß bei Raumtemperatur d.h. bei etwa 23° C bei Tageslicht und künstlicher Laborbeleuchtung, Bedingungen wie sie auch in der Praxis auftreten. NCO-Gehalt und Farbe wurden an ca. 80 %igen Lösungen untersucht.

Die Reaktivität wurde gemessen gegenüber dem Aminogruppen und Ketimingruppen enthaltenden Härter aus Beispiel 3. Zur Messung der Reaktivität wurde das jeweilige Polyisocyanat mit dem Gemisch von Toluol/Ethylacetat 1:1 auf einen Gehalt von 50 Gew.-% verdünnt und mit dem nicht weiter verdünnten Härter aus Beispiel 3 unter gutem Rühren schnell homogen vermischt. Dabei wird ein Verhältnis von NCO-Gruppen zu Amino-Gruppen (frei oder mit Keton blockiert) von 1,08 zu 1 eingehalten. Gemessen wird die Zeit, nach der die Mischung eine Viskosität von 60 000 mPas bei 23° C erreicht hat. Um eine einwandfreie Beschichtung z.B.

vo nSpaltleder zu gewährleisten (z.B. gemäß DE-A 2 637 115) darf diese Zeit erfahrungsgemäß nicht mehr als etwa 250 bis 270 sec betragen.

Tabelle 1

| Beispiel | Stabilisator | NCO-Gehalt (Gew.-%) frisch | NCO-Gehalt (Gew.-%) nach 4 Wochen | Farbe frisch | Farbe nach 4 Wochen | Reaktivität (sec) frisch | Reaktivität (sec) nach 4 Wochen | Reaktivität (sec) nach 8 Wochen |
|---|---|---|---|---|---|---|---|---|
| 5 | - (kein Zusatz) | 3,45 | 3,05 | farblos | gelb | 270 | 460 | 600 |
| 6 | Benzoylchlorid | 3,45 | 3,40 | hellgelb | braungelb | 161 | 325 | 405 |
| 7 | Isophthalylchlorid | 3,45 | 3,30 | farblos | dunkelgelb | 163 | 271 | 330 |
| 8 | p-Toluolsulfonsäure | 3,45 | 3,32 | gelbbr./ trübe | rotbraun | 154 | 450 | 503 |
| 9 | Perfluorbutansulfonsäure | 3,62 | 3,6 | farblos | int. gelb | 150 | 203 | 280 |
| 10 | Methansulfonsäure | 3,62 | 3,45 | " | gelb | 303 | 297 | 370 |
| 11 | Essigsäure | 3,45 | 3,23 | " | int. gelb | 249 | 210 | 230 |
| 12 | Dichloressigsäure | 3,62 | 3,45 | " | dunkelgelb | 180 | 297 | 340 |
| 13 | Monofluoressigsäure | 3,62 | 3,30 | schwach gelb | " | 240 | 298 | 320 |
| 14 | Ameisensäure | 3,45 | 3,25 | " | int. gelb | 287 | 309 | 450 |
| 15 | 2-Chlorpropionsäure | 3,45 | 3,40 | farblos | farblos | 230 | 256 | 270 |
| 16 | 2-Chlorpropionsäure | 3,62 | 3,55 | " | hellgelb | 205 | 238 | 261 |
| 17 | 2-Chlorpropionsäure | 3,45 | 3,33 | schwach gelb | schwach gelb | 273 | 268 | 270 |

Ergebnis:

Alle Stabilisatoren bewirken eine gute Konstanz des NCO-Gehaltes. Die Vergleichsversuche 5-14 zeigen, daß die Stabilisatoren vom Stand der Technik nicht in der Lage ist, die geforderte Stabilisierung gegen Verfärbung zu leisten. Zu einer Stabilisierung der Reaktivität sind die meisten Stabilisatoren des Standes der Technik ungeeignet. Die erfindungsgemäßen Beispiele 15, 16 und 17 zeigen in der Stabilisierung von NCO-Gehalt, Farbe und Reaktivität die besten Ergebnisse. Auch der Zusatz des Stabilisators schon während der Umsetzung (Beispiel 17) bringt bei NCO-Gehalt und Reaktivität gute Ergebnisse, die Farbe wird geringfügig verschlechtert.

Beispiele 18 bis 28

Man verfährt wie in den Beispielen 5 bis 17 und untersucht das Polyisocyanat aus Beispiel 2. Zur Stabilisierung werden wieder Stabilisatoren vom Stand der Technik und die erfindungsgemäßen eingesetzt. Bei Raumtemperatur (ca. 23° C) werden in die 80 %ige Lösung des Polyisocyanats je 200 ppm Stabilisator homogen eingearbeitet. Wie in den Beisielen 5 bis 17 werden NCO-Gehalt, Verfärbung und Reaktivität vor und nach Lagerung in farblosen Glasflaschen bei etwa 23° C untersucht. Die Reaktivität wird gegenüber dem Härter aus Beispiel 3 gemessen. Beide Lösungen werden unverdünnt eingesetzt.

Das Verhältnis von NCO-Gruppen zu Aminogruppen (frei oder mit Keton blockiert) beträgt 1,1. Gemessen wird die Zeit, nach der die Mischung eine Viskosität von 60 000 mPas erreicht hat. Diese Zeit sollte ebenfalls unter 300 sec liegen, um eine problemlose Verarbeitung auf schnellen Beschichtungsmaschinen zu ermöglichen.

T a b e l l e 2

| Beispiel | Stabilisator | NCO-Gehalt (Gew.-%) | | Farbe | | Reaktivität (sec) | |
|---|---|---|---|---|---|---|---|
| | | frisch | nach 21 Tagen | frisch | nach 21 Tagen | frisch | nach 21 Tagen |
| 18 | - (kein Zusatz) | 3,3 | 2,9 | farblos | gelb | 397 | 520 |
| 19 | Benzoylchlorid | 3,3 | 3,12 | hellgelb | gelbbraun | 238 | 743 |
| 20 | Ameisensäure | 3,28 | 2,98 | farblos | hellgelb | 273 | 315 |
| 21 | Essigsäure | 3,28 | 2,95 | " | gelb | 198 | 295 |
| 22 | p-Toluolsulfonsäure | 3,28 | 2,95 | " | dunkelgelb | 276 | 350 |
| 23 | Dichloressigsäure | 3,3 | 3,15 | " | gelb | 286 | 344 |
| 24 | 3-Chlorpropionsäureamid | 3,3 | 3,00 | " | hellgelb | 290 | 410 |
| 25 | Perfluorbutansulfonsäure | 3,3 | 3,18 | schwach gelb | gelbbraun | 203 | 246 |
| 26 | Monofluoressigsäure | 3,28 | 2,97 | farblos | dunkelgelb | 199 | 266 |
| 27 | 2-Chlorpropionsäure | 3,28 | 3,18 | " | farblos | 224 | 238 |
| 28 | 3-Chlorpropionsäure | 3,28 | 3,10 | " | " | 208 | 240 |

Ergebnis:

Im Vergleich zu allen geprüften Stabilisatoren erbringen die erfindungsgemäßen Versuche 27 und 28 auch beim aliphatischen Polyisocyanat die besten Stabilisierungsergebnisse in der Summe aller untersuchten Eigenschaften.

Anwendungsbeispiel

| | |
|---|---|
| 100 g | des Prepolymeren gemäß Beispiel 1 |
| 45,6 g | des Härters gemäß Beispiel 3 |
| 6,8 g | eines Farbpigments |
| 0,1 g | 2-Chlorpropionsäure |

werden in einer 2-Komponenten Spritzpistole der Bauart EMU 10 der Firma Maschinenfabrik Hennecke (BRD) vermischt und auf eine Matrize aufgespritzt. (Die Pistole wird mechanisch hin und her bewegt, die Matrize wird liegend senkrecht zur Bewegungsrichtung der Pistole gleichmäßig darunter fortbewegt).

Nach verschiedenen Zeitspannen, gerechnet vom Auftragen der Mischung, werden Vliesstoffe oder Spaltleder von Hand aufgelegt und durch Walzen mechanisch gleichmäßig aufgedrückt.

Die beschichteten Teile passieren dann einen auf etwa 80° C geheizten Trockenkanal. Nach 4 Minuten können die Teile von der Matrize abgezogen werden. Die Beschichtungen haben eine lederähnliche Narbung, die beim Stapeln voll erhalten bleibt, sind völlig klebfrei und lassen sich auf Schuhmaschinen schnell verarbeiten. Die beschichteten Teile sind gegen Chemikalien und Wasser beständig. Sie überstehen einen Knicktest bei -25° C mit mehr als 2000 Knickungen ohne Beschädigung.

Die Eigenschaften zeigen, daß der entstandene Polyurethanharnstoff durch den Einfluß des erfindungsgemäßen Stabilisators voll ausreagiert ist.

Führt man den gleichen Versuch mit einem Isocyanat-Prepolymer nach Beispiel 1 durch und verzichtet auf die Zugabe von 2-Chlorpropionsäure, erhält man Beschichtungen, die erst nach 8 Minuten abgezogen werden können und die erst nach einer Lagerung von 24 Stunden gestapelt und verarbeitet werden können.

## Patentansprüche

1. Polyisocyanate enthaltend 0,0001-1 Gew.% einer Carbonsäure bezogen auf Polyisocyanat der allgemeinen Formel

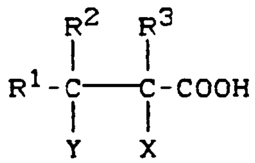

worin
$R^1$, $R^2$ und $R^3$ Wasserstoff oder $C_1$-$C_5$-Alkyl und
X und Y Wasserstoff, Chlor oder Methyl bedeuten, mit der Maßgabe, daß
X = Cl, wenn Y = H oder $CH_3$ bzw. Y = Cl, wenn X = H oder $CH_3$.

2. Polyisocyanate gemäß Anspruch 1, dadurch gekennzeichnet, daß die Carbonsäure 2-Chlorpropionsäure ist.

3. Polyisocyanate gemäß Anspruch 1, dadurch gekennzeichnet, daß die Carbonäsure 3-Chlorpropionsäure ist.

4. Polyisocyanate gemäß Anspruch 1, dadurch gekennzeichnet, daß der Gewichtsanteil der Säuren 0,001 -1,0 % beträgt.

5. Verwendung der Polyisocyanate nach Anspruch 1 für Beschichtungs-, Dichtungs- oder Klebstoffbindemittel.

## Revendications

1. Polyisocyanates renfermant 0,0001 à 1 % en poids par rapport au polyisocyanate d'un acide carboxylique de la formule générale

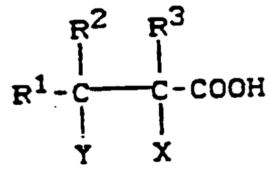

dans laquelle,
$R^1$, $R^2$ et $R^3$ correspondent à l'hydrogène ou à un radical alkyle de $C_1$ à $C_5$ , et
X et Y représentent l'hydrogène, le chlore ou un groupement méthyle, à condition que
X = Cl, si Y = H ou $CH_3$, ou Y = Cl, si X = H ou $CH_3$.

2. Polyisocyanates selon la revendication 1, caractérisés en ce que l'acide carboxylique est de l'acide 2-chloropropionique.

3. Polyisocyanates selon la revendication 1, caractérisés en ce que l'acide carboxylique est de l'acide 3-chloropropionique.

4. Polyisocyanates selon la revendication 1, caractérisés en ce que la proportion pondérale des acides atteint 0,001 à 1,0 %

5. Utilisation des polyisocyanates selon la revendication 1 pour des agents d'enduction, des agents d'obturation ou d'étanchéification et des liants dans des adhésifs.

## Claims

1. Polyisocyanates containing 0.0001 - 1% by weight, based on the polyisocyanate, of a carboxylic acid of the general formula

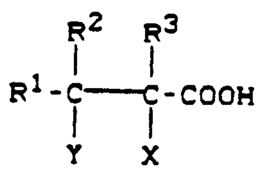

in which
$R^1$,$R^2$ and $R^3$ denote hydrogen or $C_1$-$C_5$-alkyl and
X and Y denote hydrogen, chlorine or methyl, with the proviso that
X is Cl when Y is H or $CH_3$ and Y is Cl when X is H or $CH_3$.

2. Polyisocyanates according to Claim 1, characterised in that the carboxylic acid is 2-chloropropionic acid.

3. Polyisocyanates according to Claim 1, characterised in that the carboxylic acid is 3-chloropropionic acid.

4. Polyisocyanates according to Claim 1, characterised in that the proportion by weight of the acids is 0.001 - 1.0%.

5. Use of the polyisocyanates according to Claim 1 in binders for coatings, seals and adhesives.